# EUROPÄISCHE PATENTSCHRIFT

(11) **EP 0 445 809 B1**
(45) Veröffentlichungstag und Bekanntmachung des Hinweises auf die Patenterteilung: **15.05.1996**
(21) Anmeldenummer: 91103489.0
(22) Anmeldetag: 07.03.1991
(51) Int. Cl.: A61B 5/022, A61B 5/0432, A61B 5/0205, A61B 5/0404

(54) **Blutdruckmessvorrichtung**
Device for measuring blood pressure
Dispositif de mesure de la pression sanguine

(30) Priorität: 08.03.1990 DE 4007400; 23.04.1990 DE 4012874
(43) Veröffentlichungstag der Anmeldung: 11.09.1991
(73) Patentinhaber: Müller & Sebastiani Elektronik-GmbH, D-81377 München (DE)
(72) Erfinder: Müller, Peter, W-8000 München 40 (DE); Sebastiani, Oscar, W-8000 München 5 (DE); Graf von Arnim, Thomas, Dr., W-8000 München 40 (DE)
(74) Vertreter: Dipl.-Phys.Dr. Manitz Dipl.-Ing. Finsterwald Dipl.-Ing. Grämkow Dipl.Chem.Dr. Heyn Dipl.Phys. Rotermund Morgan, B.Sc.(Phys.)

(56) Entgegenhaltungen:
- EP-A- 0 029 349
- EP-A- 0 123 313
- GB-A- 2 054 861
- US-A- 4 617 937
- US-A- 4 860 759
- US-A- 4 889 132
- US-A- 4 905 706

## Beschreibung

Die Erfindung betrifft eine Blutdruckmeßvorrichtung nach dem Oberbegriff des Patentanspruchs 1.

Ambulante Blutdruckmonitore, die eine Überwachung und Aufzeichnung der Blutdruckwerte von Patienten über längere Zeitspannen, insbesondere über 24 Stunden ermöglichen, werden in der Praxis seit längerem eingesetzt. Die Blutdruckmessung erfolgt hierbei üblicherweise in gleichbleibenden Zeitabständen, zum Beispiel jede Viertelstunde. Ein Nachteil derartiger Blutdruckmonitore ist, daß die häufig wiederholte Unterbrechung des Blutkreislaufs, insbesondere durch das Unterdrucksetzen einer Manschette, für den Patienten auf die Dauer sehr unangenehm ist.

Bei einem aus der DE-38 07 672 A1 bekannten Blutdruckmeßgerät wird der Blutdruck mittelbar über die kontinuierlich gemessene Pulswellenlaufzeit bestimmt. Dies stellt jedoch keinen vollwertigen Ersatz für eine unmittelbare Blutdruckmessung dar, da die Pulswellenlaufzeit nicht nur vom Blutdruck abhängig ist. Es ist daher weiterhin erforderlich, bei Forderung nach einer eindeutig überprüfbaren Messung den Blutdruck unter Verwendung einer Manschette unmittelbar zu messen, da derartige Manschetten auch geeicht sind.

Aus der europäischen Patentanmeldung EP 0 123 313 ist eine Blutdruckmeßvorrichtung bekannt, bei der mittels einer an einem Körperteil eines Patienten anbringbaren Manschette der Blutdruck gemessen werden kann, wobei zudem eine Vorrichtung zum Erfassen von Anormalitäten des Herzschlags vorgesehen ist. Die letztgenannte Vorrichtung bewirkt beim Auftreten von Herzschlag-Anormalitäten die Auslösung einer Blutdruckmessung.

Aus der Druckschrift GB-A-2 054 861 ist ein tragbares Gerät zur Aufzeichnung von Blutdruckmeßwerten und der Herzfrequenz eines Patienten bekannt, bei dem die aufgezeichneten Daten in einem Speichermedium abgelegt werden.

Nachteilig an den bekannten Vorrichtungen ist, daß der Patient durch die häufig wiederholten Blutdruckmessungen, welche regelmäßig eine durch das Aufblasen einer Manschette bewirkte Unterbrechung des Blutkreislaufs in einem Körperteil des Patienten bedingen, für den Patienten eine hohe Belastung darstellen.

Der Erfindung liegt daher die Aufgabe zugrunde, eine Blutdruckmeßvorrichtung der eingangs genannten Art so auszubilden, daß der Patient hinsichtlich der durchzuführenden Blutdruckmessungen entlastet wird.

Diese Aufgabe wird erfindungsgemäß durch die Merkmale des Kennzeichens des Patentanspruchs 1 gelöst.

Da auf diese Weise ein wesentlicher Anteil der gemäß dem Stand der Technik benötigten Blutdruckmessungen nicht mehr durchgeführt werden muß, ergibt sich eine ganz entscheidende Entlastung des Patienten.

Dabei kann beispielsweise festgelegt werden, daß das Unterdrücken einer Blutdruckmessung erst dann erfolgt, wenn bestimmte Parameter bzw. Parameterkombinationen mehrfach praktisch gleiche Blutdruckwerte erbracht haben, so daß eine hohe Sicherheit dafür besteht, daß durch das Unterdrücken von Blutdruckmeßvorgängen keine Verringerung der Gesamt-Aussagekraft von Meßwertaufzeichnungen entsteht. Die Unterdrückung an sich unnötiger wiederholter Blutdruckmessungen erhöht auch die Übersichtlichkeit der erzielbaren Darstellungen und erleichtert dementsprechend die Diagnose.

Eine weitere Entlastung des Patienten ergibt sich dadurch, daß die jeweiligen Blutdruckmessungen stets nur bei definierten, vorgebbaren Körperzuständen durchgeführt werden, für die sie für eine spätere Diagnose benötigt werden. Dabei kommen insbesondere kritische Körperzustände in Frage, bei denen bestimmte Grenzwerte der mittels des Langzeit-EKG-Geräts ermittelten Parameter unter- bzw. überschritten werden. Hierbei wird auch dem Umstand Rechnung getragen, daß der Blutdruck nicht nur vom Alter und der Konstitution des jeweiligen Patienten abhängig ist, sondern auch von deren psychischem und physischem Zustand. Blutdruckmessungen bei solchen Körperzuständen, die im Zusammenhang mit einer später durchgeführten Diagnose nicht von Interesse sind, werden nicht durchgeführt, so daß der Patient insgesamt weniger belastet ist.

Vorzugsweise sind auch die mittels der Langzeit-EKG-Meßeinrichtung ermittelten Parameterwerte in einem Speicher ablegbar, so daß diese Parameter zusammen mit den zugeordneten Blutdruckwerten wieder abgerufen werden können und für eine spätere Diagnose die erforderliche Zuordnung der Werte zueinander gegeben ist.

Vorteilhafterweise erfolgt die Blutdruckmessung bei mehreren unterschiedlichen, vorgebbaren Werten der mittels der Langzeit-EKG-Meßeinrichtung ermittelten Parameter bzw. bei mehreren unterschiedlichen, von vornherein festlegbaren Körperzuständen des Patienten.

Die entsprechende Sensoren aufweisende Langzeit-EKG-Meßeinrichtung ist in besonders vorteilhafter Weise zur Bestimmung solcher Parameter ausgelegt, wie sie in Form des QRS-Komplexes, der QRS-Zeitintervalle, der Herzfrequenz, der Anzahl der Extrasystolen, der ST-Veränderungen, der Arrhytmieüberwachung, des Pulses an einer Extremität, insbesondere am Ohrläppchen, der Pulswellenlaufzeit, d er Körpertemperatur, der Atemfrequenz, der Arteriengeräusche und/oder von pCO₂-Änderungen vorliegen.

Der sogenannte QRS-Komplex entspricht der Erregungsausbreitung in beiden Ventrikeln. Zur Bestimmung der Pulswellenlaufzeit (PWL) wird beispielsweise die Laufzeit der durch jeden Herzschlag verursachten Pulswelle gemessen, wobei man entweder die Zeitdifferenz zwischen der R-Zacke des Elektrokardiogramms und der Ankunft des Pulses an einer peripheren Arterie mißt oder die Zeitdifferenz zwischen zwei verschiedenen weit distal liegenden Pulsen über mechanische oder optische Aufnehmer erfaßt.

Für die jeweilige Diagnose ist es zweckmäßig, wenn die Ergebnisse der Blutdruck- bzw. EKG-Messung über eine optische Anzeige darstellbar und/oder über eine Ausgabeeinheit abrufbar sind.

Das insbesondere im Zusammenhang mit einem Blutdruckmeßgerät der genannten Art verwendbare erfindungsgemäße Langzeit-EKG-Gerät umfaßt vorteilhafterweise eine Auswerteschaltung, welche bei Auftreten vorgebbarer, eine jeweilige Blutdruckmessung erfordernder Werte der ermittelten Parameter ein entsprechendes Steuersignal für das Blutdruckmeßgerät liefert und/oder einen akkustischen oder optischen Signalgeber ansteuert. Wird beispielsweise ein entsprechender Signalton wahrgenommen, so kann anschließend der Patient oder ein anwesender Arzt die Manschette anlegen und die erforderliche Blutdruckmessung vornehmen.

Beim verwendeten Blutdruckmeßgerät ist hierbei ggf. lediglich ein eine jeweilige Blutdruckmessung veranlassende Auslöseschaltung vorzusehen, die vom Steuersignal des Langzeit-EKG-Geräts beaufschlagbar ist.

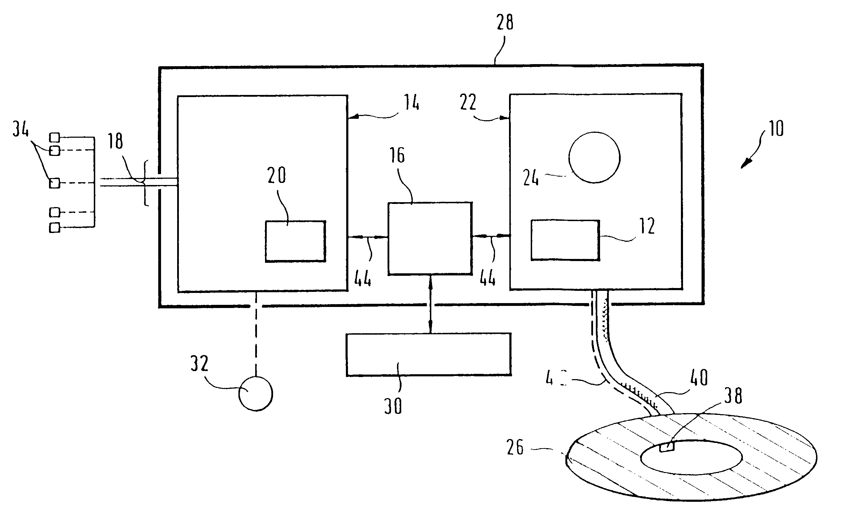

Die Erfindung wird im folgenden anhand der einzigen Figur näher erläutert.

Diese zeigt in schematischer Darstellung eine BlutdruckmeßVorrichtung 10 zur automatischen Blutdruckmessung über einen längeren Zeitraum hinweg.

Diese Blutdruckmeßvorrichtung 10 weist eine Auswerte- nd Steuerschaltung 16 mit zugeordneten Speichern 12 und 20 für die gewonnenen Meßwerte auf. Dabei werden im Speicher 12 die im Verlauf der Überwachung erfaßten Blutdruckmeßwerte gespeichert.

Die Blutdruckmeßvorrichtung 10 umfaßt außerdem dem zur unmittelbaren Blutdruckmessung erforderlichen Geräteteil 22 mit einer zur automatischen Betätigung einer Manschette 26 dienenden Pumpe 24 eine Langzeit-EKG-Meßeinrichtung 14, der der Speicher 20 zur Aufnahme von insbesondere herzfreguenzspezifischen Parametern 18 zugeordnet ist.

Die genannten Parameter 18 werden über eine Reihe von Sensoren 34 ermittelt, bei denen es sich beispielsweise um am Körper von Patienten anlegbare Elektroden handeln kann.

Die Langzeit-EKG-Einrichtung 14 ist zur Ermittlung unterschiedlichster, im einzelnen bereits erläuterter Parameter ausgelegt. Grundsätzlich ist sowohl die Messung einzelner als auch einer bestimmten Kombination von Parametern möglich, wobei das Gerät vorzugsweise so ausgebildet ist, daß bestimmte Paramter bzw. Parameterkombinationen fest vorgegeben und vom Arzt patientenabhängig ausgewählt werden können. Grundsätzlich kann aber auch der das Gerät benutzende Arzt Parameterkombinationen selbst frei vorgeben und auswählen.

Der zur automatischen Blutdruckmessung erforderliche Geräteteil 22 einschließlich der zur automatischen Betätigung der Manschette 26 dienenden Pumpe 24 ist zusammen mit der Langzeit-EKG-Meßeinrichtung 14 sowie der Auswerte- und Steuerschaltung 16 einschließlich der zugeordneten Speicher 12, 20 zu einem System 28 zusammengefaßt, das als tragbares, am Patienten anbringbares System ausgebildet ist. Eine jeweilige Blutdruckmessung einschließlich einer entsprechenden Betätigung der Manschette 26 mittels der Pumpe 24 erfolgt in Abhängigkeit davon, ob die mittels der Langzeit-EKG-Meßeinrichtung 14 ermittelten Parameter 18 vorgebbare Werte aufweisen oder nicht. Beispielsweise erfolgt eine Druckmessung, sobald diese Parameterwerte bestimmte Grenzwerte über- oder unterschreiten bzw. innerhalb bestimmter Wertebereiche liegen.

Die Blutdruckmessung wird bei mehreren unterschiedlichen, vorgebbaren Parameterwertern, d.h. bei unterschiedlichen definierten Körperzuständen durchgeführt.

Ferner umfaßt die Auswerte- und Steuerelektronik 16 eine Wiederholungserkennung, über die feststellbar ist, wann während des Meßzeitraums bestimmte Parameterwerte bzw. Kombinationen von Parameterwerten wiederholt auftreten. In diesem Falle sorgt die Elektronik nach entsprechendem Vergleich einer Mehrzahl solcher Konstellationen in Abhängigkeit von dem Vergleichsergebnis dafür, daß bei dieser bestimmten Konstellation eine nochmalige Blutdruckmessung unterbleibt, und davon auszugehen ist, daß eine weitere Messung nur nochmals den bereits bekannten Wert erbringen würde.

Die gewonnenen Ergebnisse der Blutdruck- bzw. EKG-Messung sind über eine optische Anzeige 30 darstellbar. Alternativ oder zusätzlich kann auch eine Ausgabeeinheit für einen Abruf dieser Daten vorgesehen sein. Es kann auch eine Eingabeeinheit insbesondere zur Eingabe der vorzugebenden Parameter-Vorgaben vorhanden sein.

Im vorliegenden Fall liefert das Langzeit-EKG-Gerät zur Auslösung der Blutdruckmessung über eine Leitung 44 ein Steuersignal an den Geräteteil 22 zur automatischen Durchführung der Blutdruckmessung. Wie in der Zeichnung dargestellt, wird dieses Steuersignal in diesem speziellen Fall von der Auswerte- und Steuerschaltung 16 erzeugt. Grundsätzlich ist es auch denkbar, das EKG-Gerät mit einer Auswerteschaltung zu versehen, welche beim Auftreten vorgebbarer, eine Blutdruckmessung erfordernder Parameterwerte einen akustischen oder optischen Signalgeber 32 ansteuert.

Besonders günstig kann sich im Rahmen der Erfindung die Verwendung des zeitlichen Verlaufs der Atemfrequenz sowie des Strömungsgeräuschs in Arterien als Parameter auswirken, und zwar insbesondere auch in Kombination mit weiteren bereits erwähnten Parametern.

Die Berücksichtigung der Atemfrequenz kann im Zusammenhang mit der Blutdruckmessung für den Arzt wesentliche Aufschlüsse bei der Apnoe-Behandlung erbringen, und die Auswertung des Strömungsgeräuschs in Arterien erbringt Aufschlüsse über den Gefäßzustand, so daß wiederum Größen erhalten werden, die in Verbindung mit der Blutdruckmessung sowohl für die Diagnose als auch für die Ermittlung bzw. Bildung eines den momentanen Blutdruckverlauf repräsentierenden Signals von Bedeutung sind.

Aufgrund der zeitsynchonen Erfassung einer Mehrzahl von Meßgrößen kann dem Arzt durch die entsprechende Darstellung ein wertvolles Diagnose-Hilfsmittel zur Verfügung gestellt werden, aber diese zeitsynchrone Erfassung von mehreren Meßwerten ermöglicht es auch, das Gerät mit einer ausgeprägten Lernfähigkeit auszustatten, da durch Vergleich des Verlaufs verschiedener Parameterwerte mit beispielsweise über eine geeichte Manschette durchgeführte Blutdruckmessungen eine hohe Korrelation zwischen diesen zeitlich beabstandeten Blutdruckmessungen und dem Verlauf eines Meßsignals oder einer eventuell gewichteten Kombination mehrerer Meßsignale geschaffen werden kann, so daß dann aus der entsprechenden Darstellung dieses Signalverlaufs die Blutdruckwerte praktisch kontinuierlich abgelesen und mit den übrigen zeitsynchron aufgezeichneten Meßwerten verglichen werden können.

Von Vorteil ist es ferner, den Momentanzustand des jeweiligen Patienten dadurch zu berücksichtigen, daß die zur Bildung eines dem Blutdruckverlauf entsprechenden Signals verwendeten Parameter automatisch verändert werden, wenn sich der Patient beispielsweise in einer Ruhephase, einer Aktivphase oder einer Erregungsphase befindet. Auf diese Weise ist es wiederum nicht nur möglich, die Aussagekraft zeitsynchroner Meßwertdarstellungen für den Arzt zu verbessern, sondern auch die Genauigkeit eines gebildeten kontinuierlichen Meßsignals, das den Blutdruck repräsentiert, so zu erhöhen, daß praktisch stets eine Übereinstimmung mit zeitlich beabstandeten Blutdruck-Einzelmessungen mittels geeichter Manschette erhalten wird.

## Patentansprüche

1. Blutdruckmeßvorrichtung, insbesondere zur automatischen Blutdruckmessung über einen längeren Zeitraum, bei der die jeweils erhaltenen Meßwerte in einem Speicher (12) abgelegt werden, wobei die Blutdruckmeßvorrichtung eine Langzeit-EKG-Meßeinrichtung (14) sowie eine Auswerte- und Steuerschaltung (16) umfaßt, über die eine jeweilige Blutdruckmessung in Abhängigkeit davon auslösbar ist, ob die mittels der Langzeit-EKG-Meßeinrichtung (14) ermittelten Parameter (18) vorgebbare Werte aufweisen oder nicht,
dadurch **gekennzeichnet,**
daß die Auswerte- und Steuerschaltung (16) eine Wiederholungserkennung zur Erfassung von während des Meßzeitraums mehrfach auftretenden Parameterwerten umfaßt und derart ausgelegt ist, daß beim wiederholten Auftreten bestimmter Parameterwerte eine erneute Blutdruckmessung unterbleibt.

2. Blutdruckmeßvorrichtung nach Anspruch 1,
dadurch **gekennzeichnet,**
daß die Blutdruckmessung bei mehreren unterschiedlichen Werten der mittels der Langzeit-EKG-Meßeinrichtung (14) ermittelten Parameter (18) erfolgt und vorzugsweise zusätzlich die mittels der Langzeit-EKG-Meßeinrichtung (14) ermittelten Parameter (18) in einem Speicher (20) ablegbar sind.

3. Blutdruckmeßvorrichtung nach Anspruch 1 oder 2,
dadurch **gekennzeichnet,**
daß die entsprechende Sensoren (34) aufweisende Langzeit-EKG-Meßeinrichtung (14) zur Bestimmung solcher Parameter ausgelegt ist, wie sie sich insbesondere anhand des QRS-Komplexes, der QRS-Zeitintervalle, der Herzfrequenz, der Anzahl der Extrasystolen, der ST-Veränderungen, einer Arythmieüberwachung, des Pulses an einer Extremität, insbesondere am Ohrläppchen, der Pulswellenlaufzeit (PWL), der Körpertemperatur und / oder einer pCO₂-Änderung ergeben.

4. Blutdruckmeßvorrichtung nach einem der vorhergehenden Ansprüche,
dadurch **gekennzeichnet,**
daß der zur automatischen Blutdruckmessung erforderliche Geräteteil (22) insbesondere einschließlich einer zur automatischen Betätigung einer Manschette (26) dienenden Pumpe (24) oder dergleichen zusammen mit der Langzeit-EKG-Meßeinrichtung (14) sowie der Auswerteund Steuerschaltung (16) als von einem Patienten tragbares System (28) ausgebildet ist.

5. Blutdruckmeßvorrichtung nach einem der vorhergehenden Ansprüche,
dadurch **gekennzeichnet,**
daß die Auswerte- und Steuerschaltung (16) beim Auftreten vorgebbarer, eine Blutdruckmessung erfordernder Werte der ermittelten Parameter (18) ein entsprechendes Steuersignal für ein Blutdruckmeßgerät liefert und / oder einen akustischen oder optischen Signalgeber (32) ansteuert und vorzugsweise die Ergebnisse der Blutdruck- bzw. EKG-Messung über eine optische Anzeige (30) darstellbar und / oder über eine Ausgabeeinheit abrufbar sind.

6. Blutdruckmeßvorrichtung nach einem der vorhergehenden Ansprüche,
dadurch **gekennzeichnet,**
daß als Parameter zur Auslösung einer Blutdruckmessung oder zur Erfassung von Blutdruckmeßwerten in Abhängigkeit von einer den Blutdruck repräsentierenden Meßgröße mit anschließender Speicherung der Meßwerte die insbesondere über Thermistorsensoren erfaßbare Atemfrequenz und / oder das über Tonaufnehmer erfaßbare Strömungsgeräusch in Arterien verwendet wird bzw. werden.

7. Blutdruckmeßvorrichtung nach Anspruch 6,
dadurch **gekennzeichnet,**
daß Einrichtungen zur Erfassung und Auswertung des Spektrums und / oder der Amplituden des Strömungsgeräusches in Arterien vorgesehen sind und daß Veränderungen des Spektrums und / oder der Amplituden als Parameter oder Parameterkombinationen zur Auslösung einer Blutdruckmessung mittels einer Manschette oder zur Erfassung eines von einer kontinuierlich erfaßten Meßgröße abgeleiteten, für den Blutdruck repräsentativen Meßwertes verwendet werden.

8. Blutdruckmeßvorrichtung nach einem der vorhergehenden Ansprüche,
dadurch **gekennzeichnet,**
daß die eine Erfassung und Speicherung von Blutdruckmeßwerten auslösenden Werte von Parametern oder Parameterkombinationen programmierbar sind und daß inbesondere eine automatische Veränderung der ausgewählten Parameter, Parameterkombinationen und / oder der Werte dieser Parameter in Abhängigkeit von Zustandssituationen des Patienten, insbesondere in Abhängigkeit von Ruhe-, Aktiv- und Erregungsphasen vorgesehen ist.

9. Blutdruckmeßvorrichtung nach einem der vorhergehenden Ansprüche,
dadurch **gekennzeichnet,**
daß in der Auswerteeinheit eine zeitsynchrone Darstellung einer Mehrzahl von kontinuierlich erfaßten Meßwerten, insbesondere von Herzfrequenz, Pulswellenlaufzeit, Temperatur, weiteren EKG-Größen und dergleichen vorgesehen und eine Zuordnung zwischen aktuellen Blutdruckmeßwerten und den zum Zeitpunkt der Auslösung der Blutdruckmessung vorliegenden Parameterwerten geschaffen ist.

10. Blutdruckmeßvorrichtung nach einem der vorhergehenden Ansprüche,
dadurch **gekennzeichnet,**
daß in Abhängigkeit von der Steilheit zeitlicher Veränderungen vorgebbarer Parameter oder Parameterkombinationen Blutdruckmeßwerte gebildet und gespeichert werden.

11. Blutdruckmeßvorrichtung nach einem der vorhergehenden Ansprüche,
dadurch **gekennzeichnet,**
daß durch patientenbezogenen Vergleich kontinuierlich erfaßter Parameter oder Parameterkombinationen mit Blutdruck-Einzelmessungen ein kontinuierliches Signal ausgewählt oder gebildet wird, das dem zeitlichen Blutdruckverlauf bestmöglich angenähert ist, und daß bei der Bildung des dem zeitlichen Blutdruckverlauf angenäherten kontinuierlichen Signals insbesondere verschiedene zur Bildung dieses Signals verwendete Parametergrößen unterschiedlich gewichtet werden, wobei die Gewichtung vorzugsweise in Abhängigkeit von jeweils zeitlich vorhergehenden Meßwertvergleichen variiert wird.

## Claims

1. Blood pressure measurement apparatus, in particular for the automatic measurement of blood pressure over a long period of time in which the respectively obtained measured values are stored in a memory (12), with the blood pressure measurement apparatus including a long term ECG measurement device (14) and also an evaluation and control circuit (16) via which a respective blood pressure measurement can be triggered in dependence on whether the parameters (18) derived by means of the long term ECG measurement device (14) have predeterminable values or not,
**characterized in that**
the evaluation and control circuit (16) includes repetition recognition to detect multiply occurring parameter values during the measurement time period and is laid out such that with the repeated occurrence of specific parameter values a renewed blood pressure measurement is omitted.

2. Blood pressure measurement apparatus in accordance with claim 1,
**characterized in that**
the blood pressure measurement takes place with several different values of the parameter (18) determined by means of the long term ECG measurement device (14) and the parameters (18) which are determined by means of the long term ECG measurement device are preferably additionally storable in a memory (20).

3. Blood pressure measurement apparatus in accordance with claim 1 or claim 2,
**characterized in that**
the long term ECG measurement device (14) having corresponding sensors (34) is laid out to determine parameters such as in particular result with reference to the QRS complex, the QRS time intervals, the heart rate, the number of extrasystoles, the ST changes, the monitoring of arythmia, the pulse at an extremity, in particular the earlobe, the pulse wave passage time (PWL), the body temperature and/or a pCO2 change.

4. Blood pressure measurement apparatus in accordance with one of the preceding claims,
**characterized in that**
the apparatus part (22) required for the automatic blood pressure measurement, in particular including a pump (24) serving for the automatic actuation of a cuff (26) or the like, together with the long term ECG measurement device (14) and also the evaluation and control circuit (16), is formed as a system (28) carried by a patient.

5. Blood pressure measurement apparatus in accordance with one of the preceding claims,
**characterized in that**
on the occurrence of predeterminable values of the determined parameter which necessitate a blood pressure measurement the evaluation and control circuit (16) delivers a corresponding control signal for a blood pressure measurement apparatus and/or activates an acoustical or optical signal transuser (32) with the results of the blood pressure measurement or ECG measurement preferably being portrayable via an optical display unit (30) and/or being capable of being called up via an output unit.

6. Blood pressure measurement apparatus in accordance with one of the preceding claims,
**characterized in that**
the breathing rate, which can in particular be detected by thermistor sensors, and/or the flow noise in arteries, which can be detected via acoustic pick-ups, is/are used as parameters to trigger a blood pressure measurement, or for the detection of blood pressure measurement values in dependence on a measurement parameter representing the blood pressure, with subsequent storage of the measured values.

7. Blood pressure measurement apparatus in accordance with claim 6,
**characterized in that**
means are provided for detecting and evaluating the spectrum and/or the amplitudes of the flow noise in the arteries, and in that changes of the spectrum and/or of the amplitudes are used as a parameter or parameter combinations for triggering a blood pressure measurement by means of a cuff, or for detecting a measurement value representative of the blood pressure derived from a continuously monitored measurement parameter.

8. Blood pressure measurement apparatus in accordance with one of the preceding claims,
**characterized in that**
the values of parameters or parameter combinations which trigger a detection and storage of blood pressure measurement values can be programmed, and in that in particular an automatic change of the selected parameters, parameter combinations and/or of the values of these parameters in dependence on situation states of the patient is provided, in particular in dependence on rest phases, active phases and stimulation phases.

9. Blood pressure measurement apparatus in accordance with one of the preceding claims,
**characterized in that**
a time synchronous portrayal of a plurality of continuously detected measured values, in particular of the heart rate, pulse wave transit time, temperature, further ECG parameters and the like is provided in the evaluation unit and an association is created between actual blood pressure measurement values and the parameter values present at the point in time of the triggering of the blood pressure measurement.

10. Blood pressure measurement apparatus in accordance with one of the preceding claims,
**characterized in that**
blood pressure measurement values are formed and stored in dependence on the steepness of time changes of predeterminable parameters or parameter combinations.

11. Blood pressure measurement apparatus in accordance with one of the preceding claims,
**characterized in that**
a continuous signal is selected or formed which is approximated as well as possible to the time plot of the blood pressure through a patient-related comparison of continually detected parameters or parameter combinations with individual blood pressure measurements; and in that, for the formation of the continuous signal approximated to the time plot of the blood pressure, different parameter values are in particular differently weighted in order to form this signal, with the weighting preferably being varied in dependence on respective timewise preceding measured value comparisons.

## Revendications

1. Dispositif de mesure de la pression sanguine, en particulier destiné à la mesure automatique de la pression sanguine sur une période prolongée, dans lequel les différentes valeurs mesurées obtenues sont stockées dans une mémoire (12), ledit dispositif de mesure de la pression sanguine comprenant un dispositif de mesure d'ECG Holter (14) ainsi qu'un circuit d'évaluation et de commande (16) par l'intermédiaire desquels une mesure de pression sanguine peut être déclenchée en fonction du fait que les paramètres (18) déterminés par le dispositif de mesure d'ECG Holter (14) présentent ou non des valeurs prédéfinissables, caractérisé en ce que le circuit d'évaluation et de commande (16) comprend une reconnaissance de répétition pour l'acquisition des valeurs de paramètres apparaissant plusieurs fois au cours de la période de mesure, et en ce qu'il est conçu de telle sorte qu'une nouvelle mesure de pression sanguine ne se produit pas lorsque des valeurs de paramètres déterminées apparaissent de manière répétée.

2. Dispositif de mesure de la pression sanguine selon la revendication 1, caractérisé en ce que la mesure de la pression sanguine a lieu en présence de plusieurs valeurs différentes des paramètres (18) déterminés au moyen du dispositif de mesure d'ECG Holter (14), et en ce que les paramètres (18) déterminés par le dispositif de mesure d'ECG Holter (14) peuvent de préférence être en plus stockés dans une mémoire (20).

3. Dispositif de mesure de la pression sanguine selon la revendication 1 ou 2, caractérisé en ce que le dispositif de mesure d'ECG Holter (14) présentant des capteurs (34) correspondants est conçu de manière à pouvoir déterminer des paramètres tels que ceux définis en particulier sur la base du complexe QRS, de l'intervalle de temps QRS, de la fréquence cardiaque, du nombre d'extrasystoles, des modifications du segment ST, du monitorage d'une arythmie, du pouls à une extrémité, en particulier au niveau du lobe de l'oreille, du temps de propagation de l'onde de pouls, de la température corporelle et/ou d'une modification de la pCO₂.

4. Dispositif de mesure de la pression sanguine selon l'une quelconque des revendications précédentes, caractérisé en ce que la partie d'appareil (22) nécessaire pour la mesure automatique de la pression sanguins, y compris en particulier une pompe (24) servant à actionner automatiquement un brassard (26) ou similaire, est formée en association avec le dispositif de mesure d'ECG Holter (14) et avec le circuit d'évaluation et de commande (16) comme un système (28) portable par le patient.

5. Dispositif de mesure de la pression sanguine selon l'une quelconque des revendications précédentes, caractérisé en ce que le circuit d'évaluation et de commande (16) fournit, en cas d'apparition de valeurs prédéfinissables des paramètres déterminés (18) nécessitant une mesure de la pression sanguine, un signal de commande correspondant à un appareil de mesure de la pression sanguine et/ou excite un transmetteur de signal acoustique ou optique (32), et en ce que les résultats de la mesure de pression sanguine ou de l'ECG peuvent être de préférence présentés sur un affichage optique (30) et/ou téléchargés par l'intermédiaire d'une unité de sortie.

6. Dispositif de mesure de la pression sanguine selon l'une quelconque des revendications précédentes, caractérisé en ce que le paramètre utilisé pour déclencher une mesure de la pression sanguine ou l'acquisition de valeurs de pression sanguine mesurées en fonction d'une grandeur de mesure représentant la pression sanguine, suivie de la mémorisation des valeurs mesurées, est la fréquence respiratoire détectable par l'intermédiare de capteurs à thermistance et/ou le bruit circulatoire dans les artères détectable par l'intermédiaire de capteurs acoustiques.

7. Dispositif de mesure de la pression sanguine selon la revendication 6, caractérisé en ce que des dispositifs d'acquisition et d'évaluation du spectre et/ou des amplitudes du bruit circulatoire dans les artères sont prévus et en ce que les modifications du spectre et/ou des amplitudes sont utilisées comme paramètres ou combinaisons de paramètres pour déclencher une mesure de la pression sanguine au moyen d'un brassard ou pour l'acquisition d'une valeur de mesure dérivée d'une grandeur mesurée acquise de manière continue et représentative de la pression sanguine.

8. Dispositif de mesure de la pression sanguine selon l'une quelconque des revendications précédentes, caractérisé en ce que les valeurs des paramètres ou combinaisons de paramètres déclenchant l'acquisition et la mémorisation des valeurs de pression sanguine mesurées sont programmables, et en ce qu'il est prévu en particulier une modification automatique des paramètres, des combinaisons de paramètres et/ou des valeurs de ces paramètres choisis en fonction des situations de l'état du patient, notamment en fonction des phases de repos, d'activité et d'excitation.

9. Dispositif de mesure de la pression sanguine selon l'une quelconque des revendications précédentes, caractérisé en ce qu'il est prévu dans l'unité d'évaluation une pluralité de valeurs de mesure acquises en continu, notamment des valeurs de fréquence cardiaque, de temps de propagation de l'onde de pouls, de température, d'autres grandeurs ECG et similaires, et en ce qu'il est établi une correspondance entre les valeurs de pression sanguine effectivement mesurées et les valeurs des paramètres présentes au moment du déclenchement de la mesure de pression sanguine.

10. Dispositif de mesure de la pression sanguine selon l'une quelconque des revendications précédentes, caractérisé en ce que des valeurs de pression sanguine mesurées sont formées et mémorisées en fonction de la raideur de la pente de modification dans le temps de paramètres ou combinaisons de paramètres prédéfinissables.

11. Dispositif de mesure de la pression sanguine selon l'une quelconque des revendications précédentes, caractérisé en ce que un signal continu approchant le mieux possible la courbe de pression sanguine dans le temps est sélectionné ou formé en comparant pour un même patient des paramètres ou combinaisons de paramètres acquis en continu avec des mesures de pression sanguine isolées, et en ce que, pour la formation du signal continu approchant le mieux possible la courbe de pression sanguine dans le temps, différentes grandeurs de paramètres utilisées pour former ce signal sont pondérées différemment et on fait de préférence varier la pondération en fonction des comparaisons respectives des valeurs mesurées effectuées antérieurement.
